# EUROPÄISCHE PATENTSCHRIFT

(11) **EP 0 797 456 B1**
(45) Veröffentlichungstag und Bekanntmachung des Hinweises auf die Patenterteilung: **21.02.2007**
(21) Anmeldenummer: 95905016.2
(22) Anmeldetag: 16.12.1994
(51) Int. Cl.: A61K 49/04, A61K 49/18, A61K 31/28, A61K 33/24, A61K 49/00, A61P 35/00

(54) **ANTITUMORMITTEL, ENTHALTEND EIN ZYTOSTATIKUM UND EIN KONTRASTMITTEL**
ANTICANCER DRUG WITH A CYTOSTATIC AND A CONTRASTING AGENT
MEDICAMENT ANTICANCEREUX CONTENANT UN AGENT CYTOSTATIQUE ET UN AGENT CONTRASTANT

(43) Veröffentlichungstag der Anmeldung: 01.10.1997
(73) Patentinhaber: MAX-DELBRÜCK-CENTRUM FÜR MOLEKULARE MEDIZIN, 13125 Berlin (DE)
(72) Erfinder: RESZKA, Regina, D-16341 Schwanebeck (DE); POHLEN, Uwe, D-10625 Berlin (DE); STILLER, Detlef, D-10965 Berlin (DE); BERGER, Gerd, D-10785 Berlin (DE); LIPPMANN, Matthias, D-12205 Berlin (DE)
(74) Vertreter: Baumbach, Friedrich
(86) Internationale Anmeldenummer: PCT/DE1994/001524
(87) Internationale Veröffentlichungsnummer: WO 1996/018421

(56) Entgegenhaltungen:
- WO-A-92/17214
- DE-A- 4 341 478
- CHEMICAL ABSTRACTS, vol. 120, no. 20, 1994 Columbus, Ohio, US; abstract no. 253255, KAWASHIGE, MAKOTO ET AL 'Preparation of carboplatin -Lipiodol emulsion' & KYUSHU YAKUGAKKAI KAIHO, VOL. 47, SEITEN 89-95, 1993
- CANCER CHEMOTHER PHARMACOL, 1994, , VOL. 33, SUPPL, PAGE(S) S74-S78, ICHIDA T ET AL 'Therapeutic effect of a CDDP-epirubicin-Lipiodol emulsion on advanced hepatocellular carcinoma.'
- GAN TO KAGAKU RYOHO, AUG 1988, VOL. 15, NO. 8 PART 2, PAGE(S) 2549-56, ITO K ET AL 'Preoperative chemoembolization of iopamiron-solved adriamycin and lipiodol on hepatocellular carcinoma--clinicopathological study of ten surgical cases]'
- J JPN SOC CANCER THER, 1988, VOL. 23, NO. 3, PAGE(S) 673-681., SHIMIZU T ET AL 'CLINICAL EVALUATION FOR ADVANCED HEPATOCELLULAR CARCINOMAS WITH INTRAARTERIAL INFUSION THERAPY USING POLYSACCHARIDE SOLUTION AS A CARRIER OF ANTICANCER DRUGS'
- TEDER H.; JOHANSSON C.-J.: 'THE EFFECT OF DIFFERENT DOSAGES OF DEGRADABLE STARCH MICROSPHERES SPHEREX) ON THE DISTRIBUTION OF DOXORUBICIN REGIONALLY ADMINISTERED TO THE RAT' ANTICANCER RESEARCH Bd. 13, Nr. 6, 01 November 1993, Seiten 2161 - 2164, XP000615554
- LORELIUS L.E. ET AL: 'ENHANCED DRUG RETENTION IN VX2 TUMORS BY USE OF DEGRADABLE STARCH MICROSPHERES' INVESTIGATIVE RADIOLOGY Bd. 19, Nr. 3, 01 Mai 1984, Seiten 212 - 215, XP000615557
- CZEJKA M.J.; JAEGER W.; SCHUELLER J.: 'PHARMACOKINETICS OF MITOMYCIN C IN PATIENTS AFTER BOLUS INJECTION AND CHEMOBOLISATION OF THE HEPATIC ARTERY WITH SPHEREX STARCH PARTICLES' EUROPEAN JOURNAL OF DRUG METABOLISM AND PHARMACOKINETICS Bd. 17, Nr. 2, 01 April 1992, Seiten 85 - 87, XP000615555
- MERICA G.A.; OWENS L.: 'Lollipops, liposomes, and starch microspheres: New drug delivery mechanisms' P AND T, CORE MEDICAL JOURNALS, LAWRENCEVILLE, NJ, US Bd. 19, Nr. 7, Juli 1994, Seite 728, XP008039889

## Beschreibung

Die Erfindung betrifft ein Mittel zur Antitumortherapie, seine Herstellung und seine Verwendung, insbesondere zur Therapie nichtresektabler primärer und sekundärer Lebertumoren. Anwendungsgebiete der Erfindung sind die Medizin und die pharmazeutische Industrie.

In der Antitumortherapie nichtresektabler Lebertumoren hat sich die regionale Zytostatikaapplikation in Verbindung mit flußverzögernden Substanzen zur Erhöhung der lokalen Zytostatikakonzentration am Tumor als sinnvoll erwiesen. Besonders ist eine Erhöhung der Zytostatikakonzentration am Tumor gemäß der Dosis-Wirkungsbeziehung, wie von Link sowie Collins beschrieben, unter gleichzeitiger Schonung des umgebenden Leberparenchyms anzustreben. Ziel ist es hierbei, die oben beschriebenen Wirkungen am Tumor zu erreichen und gleichzeitig die systemischen Nebenwirkungen zu minimieren. Dieses Prinzip wird in der Chemoembolisation schon seit Jahren verwirklicht.

Bei der Anwendung dieser Therapieform ergeben sich verschiedene Probleme. Die Darstellung der vollständigen Embolisation konnte bisher nur indirekt über verschiedene Methoden erreicht werden. Dabei wurde intermittierend zur Applikation des Chemoembolisats jodhaltiges Kontrastmittel injiziert, bis sich ein retrograder Kontrastmittelflow einstellt; eine weitere Methode bediente sich der Zugabe von Lipoidol, welche als jodhaltige Fettbläschen den hepatofugalen bzw. hepatopedalen Fluß anzeigen.

Die indirekte Methode hat den Nachteil, daß ein retrogrades Abfließen des Zytostatika-Embolisatgemisches nicht immer bemerkt wird, wodurch gefährliche Komplikationen (z.B. Magen-/Darmnekrosen) auftreten können. Eine Anwendbarkeit in Einrichtungen mit einfacher Röntgengeräteausstattung ist daher nur bedingt möglich.

In der Literatur sind verschiedene Kombinationen von Cytostatika mit Kontrastmitteln oder Kombinationen von Cytostatika mit Stärkepartikeln beschrieben worden.

Die gleichzeitige Verabreichung des platinhaltigen Antikrebsmittels CDDP und eines jodhaltigen Kontrastmittels (Kawashige et al. Preparation of carboplatin-Lipiodol emulsion. Chemical Abstracts 120, 536ff (1993)) mit dem zähflüssigen Mohnöl Lipiodol hat bereits gezeigt, daß dadurch die Resorption von Cytostatika in

Zellgewebe von Tumoren und somit die Prognose der Patienten verbessert werden kann. Nachteilig an dieser Wirkstoffzusammensetzung ist jedoch, daß ein großer Teil des CDDP innerhalb der Emulsionspartikel verbleibt (Ichida et al. Therapeuic effect of a CDDP-epirubicin-Lipiodol emulsion on advanced hepatocellular carcinaoma. Cancer Chemother. Pharmacol. 33, 74-78 (1994)). In einer anderen Veröffentlichung wurde eine Kombination von zwei unterschiedlichen Cytostatika mit einem Kontrastmittel und Lipiodol experimentell untersucht (Ito et al. Preoperative chemoembolization of iopamiron-solved adriamycin and lipiodol on hepatocellular carcinoma--clinicopathological study of ten surgical cases. Gan To Kagaku Ryoho 15, 2549-56 (1988)). In der Literatur ist außerdem gezeigt worden, daß Polysaccharidlösungen geeignete Träger für Cytostatika sein können (Shimizu et al. Clinical evaluation of advanced hepatocellular carcinomas with intraarterial infusion therapy using polysaccharide solution as a carrier of anticancer drugs. Nippon Gan Chiryo Gakkai Shi 23, 673-681 (1988)). Weiterhin wurde auch beschrieben, daß Spherex^{®} Stärkepartikel die Aufnahme von Cytostatika in Tumorgewebe fördern können (Teder et al. The effect of different dosages of degradable starch microspheres (Spherex) on the distribution of doxorubicin regionally administered to the rat. Anticancer Res. 13, 2161-2164 (1993) und Lorelius et al. Enhanced drug retention in VX2 tumors by use of degradable starch microspheres. Invest Radiol. 19, 212-215 (1984)). Der Embolisationseffekt von Stärkepartikeln ist darüber hinaus in Kombination mit dem Gammastrahler ^{99m}Tc beschrieben worden (Czejka et al. Pharmacokinetics of mitomycin C in patients after bolus injection and chemobolisation of the hepatic artery with Spherex starch particles. Eur J Drug Metab Pharmacokinet. 17, 85-87 (1992)). Eine weitere Veröffentlichung hat die Kombination von Spherex^{®} Stärkepartikeln mit dem Cytostatikum Cysplatin vorgestellt (Merica et al. Lollipos, Liposomes, and starch microsphees: new drug delivery mechanisms. P & T Core Medical Journals 19, 728 (1994)).

Die Erfindung hat das Ziel, die bei der Chemoembolisation von Tumoren auftretenden Nachteile zu vermeiden. Ihr liegt die Aufgabe zugrunde, ein optimales Embolisat zu entwickeln, welches nicht nur die durch die jeweilige Gefäßstruktur des Tumors bedingte Wirkung zeigt, sondern auch direkt und zuverlässig im Tumormonitoring dargestellt werden kann.

Das erfindungsgemäße Mittel ist durch
- lyophilisierte Stärkepartikel in Kombination mit
- Cytostatika, gelöst in
- jod-, gadolinium- oder magnetithaltigen Kontrastmitteln gekennzeichnet.
   Der wesentliche Bestandteil des Mittels sind die lyophilisierten Stärkepartikel. Gemäß Anspruch 1 sind diese Partikel der Größe 40-90 µm, sie werden in physiologischer Kochsalzlösung bis zur Konzentration von 50-70 mg/ml gelöst und danach in an sich bekannter Weise lyophilisiert.
   Als Cytostatikum können sämtliche bekannten Cytostatika eingesetzt werden. Gut geeignet ist u.a. Carboplatin, welches in ebenfalls lyophilisierter Form mit der lyophilisierten Stärke vermischt wird.
   Als jodhaltige Kontrastmittel dienen flüssige jodhaltige Verbindungen, bevorzugt ein- oder mehrfach jodierte Phenylderivate. Geeignet sind u. a. Iopromid, Ioxitalamat, Ioxaglat, Iopamidol, Iohexol, Iotralon, Metrizamid oder Ultravist. Das Kontrastmittel dient gleichzeitig als Lösungsmittel für das Gemisch der Lyophilisate.
   Für die Magnetresonanztomographie (MRT) werden entweder gadolinium- oder magnetithaltige Kontrastmittel eingesetzt.
   Die Komponenten sind bevorzugt in folgenden Mengenverhältnissen im Mittel enthalten: 30-90 mg lyophilisierte Stärkepartikel werden mit den benötigten Mengen Cytostatikum vermischt und nachfolgend in 3-6 ml Kontrastmittel gelöst. Man kann die angegebene Menge der lyophilisierten Stärkepartikel auch erst im Kontrastmittel lösen und danach die therapeutisch notwendige Menge Cytostatikum zusetzen.
   Zur Herstellung von lyophilisierten Stärkepartikeln wird bevorzugt vom handelsüblichen Embolisat der Firma Kabi-Pharmazia "Spherex" ausgegangen. Dieses Produkt wird im Dialyseschlauch gegen steriles Aqua bidest für 36 Stunden mit dreifachem Wasserwechsel dialysiert. Danach wird das Material mit einer sterilen Pipette aus dem Dialyseschlauch entnommen und in einem sterilen Kunststoffgefäß bei -70°C eingefroren. Das kalte Gefäß wird in die Gefriertrocknung gebracht und 24 Stunden im Hochvakuum getrocknet.
   Zur Verwendung des Mittels und seine Auswirkungen werden folgende Ausführungen gemacht.
   Die Anforderungen an ein Embolisat zur Tumortherapie richtet sich nach der Gefäßstruktur des Tumors, d.h. die Partikel sollten geeignet groß sein, um den Tumor über eine Embolisation möglichst peripherer Anteile des Tumorgefäßbettes mitsamt Zytostatikum zu erreichen. Die hierfür optimale Partikelgröße liegt zwischen 40 und 90 µm. Eine Abweichung von dieser o.g. Größe nach oben bewirkt eine Stase der zuführenden Tumorgefäße, so daß das Zytostatikum nicht in optimal hoher Konzentration den Tumor erreicht. Dieses ist bedingt durch einen peripheren Blutzustrom über die Eröffnung der arteriovenöser Shunts und den damit verbundenen Verdünnungseffekt für das Zytostatikum. Eine noch geringere Partikelgröße kann zu multiplen systemischen Embolisationen, beispielsweise Lungenembolien führen.
   Die erfindungsgemäß lyophilisierten Stärkepartikel zeigen eine Embolisationsdauer zwischen 20 und 60 Minuten. Eine Intervalltherapie mit diesem kurzwirksamen Embolisat hat sich als vorteilhaft gegenüber langwirksamen Embolisaten erwiesen. Bei der Desarterialisierung (Bengmark, usw.) kommt es zu einem rasch einsetzenden angioproliferativen Effekt, so daß innerhalb von 48 Stunden eine Neovaskularisierung am Tumor stattfindet und somit ein erneuter Therapieansatz erschwert wird. Ähnliche Effekte sieht man auch bei der Anwendung lang wirksamer Embolisate mit Thrombose der zuführenden Tumorgefäße und
   Tumorgefäßanschluß an z.B. Zwerchfell, großes Netz, usw., die eine weitere Therapie limitieren.
   Mit dem neuen Mittel und seiner Anwendung sind folgende Vorteile verbunden
- ohne Zuhilfenahme indirekter Methoden wird unter Röntgendurchleuchtung eine suffiziente Embolisation direkt dargestellt, wobei sich der Tumor mit seinen Blutgefäßen als stehendes Bild abbildet
- unter Verwendung von gadolinium- oder magnetithaltigen Kontrastmitteln in Kombination mit flußkodierten Meßsequenzen läßt sich die Embolisation auch mit Hilfe der Magnetresonanztomographie darstellen
- die erzielbare Zytostatikakonzentration im Tumorgewebe wird erheblich gegenüber anderen Darreichungsformen gesteigert(bis zum Faktor 20)
- die Applikation wird vereinfacht bei gleichzeitiger Erhöhung der Sicherheit (Vermeiden einer retrograden Fehlperfusion).

Damit kann diese Therapieform einer breiten Anwendung, auch außerhalb spezieller Therapiezentren, zugänglich gemacht werden.

Die Erfindung soll nachfolgend durch ein Ausführungsbeispiel näher erläutert werden.

### Ausführungsbeispiel

Männlichen Chinchilla-Kaninchen mit einem in die Leber implantierten VX-"-Tumor von 2 cm Durchmesser wurde ein Portsystem in die A.gastroduodenalis implantiert. Ober dieses System erhielten die Tiere nach festgelegtem Schema entweder das erfindungsgemäße Therapeutikum oder ein Gemisch aus gleichen Dosen der handelsüblichen Form. Dies beinhaltete jeweils 60 mg Spherex und 50 mg Carboplatin mit 5 ml eines 300 mg/ml jodhaltigen Kontrastmittels (Ultravist 300, Schering). Zu den festgelegten Zeitpunkten (15, 30, 60, 120, 240 min) wurden die Tiere getötet und die Zytostatikakonzentration in verschiedenen Geweben (Tumor, Leber, Milz, Nieren, Serum) analytisch unter Verwendung der Atomabsorptionsspektroskopie ermittelt. Die Konzentration des Cytostatikums im Tumorgewebe war bis zum Faktor 20 gesteigert.

Die Applikation des neu entwickelten Mittels konnte problemlos direkt unter Röntgendurchleuchtung beobachtet werden, wobei sich die allmähliche Aufsättigung des Tumorgefäßbettes bei stehenden Bildern von der Peripherie bis zum Gefäßstamm über die gesamte Phase der Embolisation darstellt und während der gesamten Dauer des Gefäßverschlusses als stehendes Bild nachvollziehbar war. Ebenso konnte die einsetzende Reperfusion dokumentiert werden.

## Patentansprüche

1. Mittel zur Antitumortherapie, **gekennzeichnet durch**
- lyophilisierte Stärkepartikel, die **durch** Lyophilisieren von Stärkepartikeln der Größe 40-90 µm, die in physiologischer Kochsalzlösung in der Konzentration von 5-70 mg/ml gelöst sind, erhalten wurden, kombiniert mit
- einem oder mehreren Cytostatika und gelöst in
- jod-, gadolinum- oder magnetithaltigen Kontrastmitteln.

2. Mittel nach Anspruch 1, **dadurch gekennzeichnet, daß** es 30-90 mg lyophilisierte Stärkepartikel und 5-100 mg lyophilisiertes Cytostatikum in 3-6 ml Kontrastmittel enthält.

3. Mittel nach Anspruch 1, **dadurch gekennzeichnet, daß** es Carboplatin als Cytostatikum enthält.

4. Mittel nach Anspruch 1, **dadurch gekennzeichnet, daß** es ein- oder mehrfach jodierte Phenylderivate als jodhaltiges Kontrastmitel enthält, vorzugsweise Iopromid^{®}, Ioxitalamat^{®}, Ioxaglat^{®}, Iopamidol^{®}, Iohexol^{®}, Iotralon^{®}, Metrizamid^{®} oder Ultravist^{®}.

5. Mittel nach Anspruch 1, **dadurch gekennzeichnet, daß** es Gadolinum- oder Magnetitderivate als Kontrastmittel enthält.

6. Verfahren zur Herstellung des Mittels, **dadurch gekennzeichnet, daß** 30-90 mg lyophilisierte Stärkepartikel in 3-6 ml Kontrastmittel gelöst werden und danach die therapeutisch notwendige Menge Cytostatikum zugesetzt wird.

7. Verwendung der Mittel gemäß der Ansprüche 1-5 zur Herstellung von Arzneimitteln zur Antitumortherapie, insbesondere zur Therapie von Lebermetastasen.

## Claims

1. Agent for anti-tumour therapy, wherein
- there exist lyophilised starch particles obtained by lyophilising starch particles of a size of 40-90 µm dissolved in a physiological saline solution with a concentration of 5-70 mg/ml combined with
- one or more cytostatics(s) and dissolved in
- contrast media containing iodine, gadolinum or magnetite.

2. Agent according to Claim 1, wherein it contains 30-90 mg of lyophilised starch particles and 5-100 mg of lyophilised cytostatics in 3-6 ml of contrast medium.

3. Agent according to Claim 1, wherein it contains Carboplatin as a cytostatic.

4. Agent according to Claim 1, wherein it contains singly or multiply iodised phenyl derivates, preferably Iopromid®, Ioxaglat®, Iohexol®, Iotralon®, Metrizamid® or Ultravist®, as a contrast medium containing iodine.

5. Agent according to Claim 1, wherein it contains gadolinum or magnetite derivates as a contrast medium.

6. Method for the production of the agent, wherein 30-90 mg of lyophilised starch particles are dissolved in 3-6 ml of contrast medium, after which the therapeutically necessary quantity of cytostatic is added.

7. Use of the agent according to Claims 1-5 for production of drugs for anti-tumour therapy, in particular for the therapy of hepatic metastases.

## Revendications

1. Produit destiné au traitement anticancereux, **se caractérisant par**
- des particules d'amidon lyophilisées qui ont été obtenues par lyophilisation de particules d'amidon de 40-90 µm qui sont dissoutes dans une solution de chlorure de sodium physiologique d'une concentration de 5 à 70 mg/ml, combinées avec .- un ou plusieurs cytostatiques et dissoutes dans
- des substances de contraste contenant de l'iode, du Gadolinum ou de la magnétite.

2. Produit selon la revendication 1, **se caractérisant par le fait qu'**il contient 30-90 mg de particules d'amidon lyophilisées et 5-100 mg de cytostatique lyophilisé dans 3-6 ml de substance de contraste.

3. Produit selon la revendication 1, **se caractérisant par le fait qu'**il contient du Carboplatine en tant que cytostatique.

4. Produit selon la revendication 1, **se caractérisant par le fait qu'**il contient des dérivés phényliques iodés une fois ou plusieurs fois en tant que substance de contraste contenant de l'iode, notamment Iopromid®, Ioxitalamat®, Ioxaglat®, Iopamidol®, Iohexol®, Iotralon®, Metrizamid® ou Ultravist®.

5. Produit selon la revendication 1, **se caractérisant par le fait qu'**il contient des dérivés de Gadolinum ou de magnétite en tant que substance de contraste.

6. Procédé de fabrication du produit, **se caractérisant par le fait que** 30-90 mg de particules d'amidon lyophilisées sont dissoutes dans 3-6 ml de substance de contraste et que la quantité thérapeutique nécessaire en cytostatiques y est ajoutée ensuite.

7. Emploi du produit selon les revendications 1-5 pour la fabrication de médicaments destinés au traitement anticancereux, en particulier le traitement des métastases du foie.
